# EUROPEAN PATENT SPECIFICATION

(11) **EP 3 337 818 B1**
(45) Date of publication and mention of the grant of the patent: **18.03.2026**
(21) Application number: 16754251.3
(22) Date of filing: 11.08.2016
(51) Int. Cl.: C07K 16/06, C07K 16/22, C07K 16/28, C07K 16/32, C07K 16/36

(54) **AFFINITY CHROMATOGRAPHY PURIFICATION WITH LOW CONDUCTIVITY WASH BUFFER**
AFFINITÄTSCHROMATOGRAFIEREINIGUNG MIT WASCHPUFFER MIT NIEDRIGER LEITFÄHIGKEIT
PURIFICATION PAR CHROMATOGRAPHIE D'AFFINITÉ AVEC UN TAMPON DE LAVAGE À FAIBLE CONDUCTIVITÉ

(30) Priority: 21.08.2015 EP 15181902
(43) Date of publication of application: 27.06.2018
(73) Proprietor: F. Hoffmann-La Roche AG, 4070 Basel (CH)
(72) Inventor: FALKENSTEIN, Roberto, 80689 Munich (DE); ROSENBERG, Eva, 82064 Straßlach-Dingharting (DE); BIALAS, Agathe, 82404 Sindelsdorf (DE); WILLMANN, Steffen, 80687 München (DE)
(74) Representative: Burger, Alexander
(86) International application number: PCT/EP2016/069162
(87) International publication number: WO 2017/032610

(56) References cited:
- EP-A1- 1 561 756
- WO-A1-2015/024896
- W. SCHAEFER ET AL: "Immunoglobulin domain crossover as a generic approach for the production of bispecific IgG antibodies", PROCEEDINGS OF THE NATIONAL ACADEMY OF SCIENCES OF THE UNITED STATES OF AMERICA, vol. 108, no. 27, 20 June 2011 (2011-06-20), US, pages 11187 - 11192, XP055563756, ISSN: 0027-8424, DOI: 10.1073/pnas.1019002108
- AMARAL J. ET AL: "Study on the scale-up of human IgG3 purification using protein A affinity chromatography", BIOSEPARATION, vol. 10, no. 4-5, 1 May 2001 (2001-05-01), NL, pages 139 - 143, XP093308880, ISSN: 0923-179X, Retrieved from the Internet <URL:https://link.springer.com/article/10.1023/A:1016353419499/fulltext.html> DOI: 10.1023/A:1016353419499
- ANONYMOUS: "Protein A/G Affinity", 29 August 2025 (2025-08-29), XP093309198, Retrieved from the Internet <URL:https://www.bio-rad.com/en-nl/applications-technologies/protein-g-affinity?ID=LUSMML97Q#:~:text=The%20affinity%20of%20protein%20A,but%20poorly%20to%20IgG>
- RITZEN ET AL: "Endotoxin reduction in monoclonal antibody preparations using arginine", JOURNAL OF CHROMATOGRAPHY B: BIOMEDICAL SCIENCES & APPLICATIONS, ELSEVIER, AMSTERDAM, NL, vol. 856, no. 1-2, 27 August 2007 (2007-08-27), pages 343 - 347, XP022215625, ISSN: 1570-0232, DOI: 10.1016/J.JCHROMB.2007.06.020

## Description

The present invention generally relates to the field of purification of polypeptides. The present invention in particular relates to the improvement of multi-column purification processes which are used in the purification of antibodies.

### Background of the Invention

Proteins and especially immunoglobulins play an important role in today's medical portfolio. For human application every therapeutic protein has to meet distinct criteria. To ensure the safety of biopharmaceutical agents to humans by-products accumulating during the production process have to be removed especially. To fulfill the regulatory specifications one or more purification steps have to follow the manufacturing process. Among other things, purity, throughput, and yield play an important role in determining an appropriate purification process.

Different methods are well established and widespread used for protein purification, such as affinity chromatography (e.g. protein A or protein G affinity chromatography, single chain Fv ligand affinity chromatography), ion exchange chromatography (e.g. cation exchange (sulfopropyl or carboxymethyl resins), anion exchange (amino ethyl resins) and mixed-mode ion exchange), thiophilic adsorption (e.g. with beta-mercaptoethanol and other SH ligands), hydrophobic interaction or aromatic adsorption chromatography (e.g. with phenyl-sepharose, aza-arenophilic resins, or m-aminophenylboronic acid), metal chelate affinity chromatography (e.g. with Ni(II)- and Cu(II)-affinity material), size exclusion chromatography, and electrophoretical methods (such as gel electrophoresis, capillary electrophoresis).

For the purification of recombinantly produced immunoglobulins often a combination of different column chromatography steps is employed. During the purification non-immunoglobulin contaminants such as host cell protein and host cell DNA as well as endotoxins and viruses are depleted. Therefore, generally an affinity chromatography step, like protein A affinity chromatography is followed by one or more additional separation steps. In general, high conductivity buffers are described to be employed in wash steps of affinity chromatrography methods. Due to e.g. high salt concentrations, further process steps may be necessary in the transition from one to another chromatography/separation step. High conductivity values in eluates from one chromatography step may have unfavorable effects on the subsequent chromatography step.

In US 6,127,526 a method for purifying proteins by Protein A chromatography is described which comprises the steps of: (a) adsorbing the protein to Protein A immobilized on a solid phase comprising silica or glass; (b) removing contaminants bound to the solid phase by washing the solid phase with a hydrophobic electrolyte solvent; and (c) recovering the protein from the solid phase.

In WO2011/038894 a protein A chromatography method with a pronounced depletion of host cell protein and DNA by specific wash steps prior to the recovery of the immunoglobulin from the protein A chromatographic material is reported.

In WO2013/177118 compositions and methods for the isolation and purification of antibodies from a sample matrix are reported.

In WO2013/033517 methods for separating a polypeptide of interest (such as an antibody) from a virus are reported.

A method for purifying a protein, including one or more chromatographic processes, in which an amino acid; or a dipeptide, an oligopeptide, or a polyamino acid thereof is included in a buffer solution used in at least one chromatographic process (equilibration buffer, wash buffer, and elution buffer), thereby purifying a high-purity protein with a very small quantity of the impurity (e.g., polymers or host cell proteins) is reported in EP2583973.

In EP 1561756 a method of purifying proteins is reported.

The separation of bispecific antibodies and bispecific antibody production side products using hydroxyapatite chromatography is reported in WO2015/024896.

Ritzen et al. (J Chromatogr B Analyt Technol Biomed Life Sci, 2007, Vol. 856, pages 343-347) report endotoxin reduction in monoclonal antibody preparations using arginine. It also reports on the use of additional wash steps (e.g. with a 20 mM phosphate buffer (pH 7.3)) in antibody purification to lower the conductivity.

### Summary of the Invention

The scope of the invention is defined by the claims.

Herein is reported a method for the production of a bispecific antibody by purifying the bispecific antibody with an affinity chromatography step as defined in the claims, followed by one ore more additional separation steps.

In more detail it has been found that by the method of the current invention which uses a low conductivity aqueous solution in a wash step of an affinity chromatography prior to the recovery of an antibody from the chromatographic material, the purification process of an antibody can be improved by obviating the need for further process steps before loading the eluate to the next column/chromatographic material and/or the content of specific host cell proteins can be reduced. For example, process steps like dilution or filtration of a protein A affinity chromatography eluate can be omitted.

One aspect as reported herein is a method for producing a bispecific antibody comprising a first antigen-binding site that specifically binds to a first antigen and a second antigen-binding site that specifically binds to a second antigen comprising the following steps
a) cultivating a cell comprising a nucleic acid encoding the bispecific antibody,
b) recovering the bispecific antibody from the cell or the cultivation medium,
c) contacting the bispecific antibody with a protein A affinity chromatography material,
d) washing the protein A affinity chromatography material with a low conductivity aqueous solution, wherein the low conductivity aqueous solution has a conductivity value of from 0.03 µS/cm to 0.5 mS/cm and wherein the low conductivity aqueous solution has a pH of about 7 or higher, and additionally washing the protein A affinity chromatography material with a medium conductivity aqueous solution before or after washing the protein A affinity chromatography material with a low conductivity aqueous solution, wherein the medium conductivity aqueous solution has a conductivity value of from more than 0.5 mS/cm to less than 20 mS/cm,
e) recovering the bispecific antibody from the protein A affinity chromatography material,
f) performing a further chromatography step
and thereby producing the bispecific antibody.

One aspect as reported herein is a method for purifying a bispecific antibody comprising a first antigen-binding site that specifically binds to a first antigen and a second antigen-binding site that specifically binds to a second antigen from a sample comprising the steps of
a) providing a sample comprising the bispecific antibody,
b) applying the sample comprising the bispecific antibody to a protein A affinity chromatography material,
c) washing the protein A affinity chromatography material with a low conductivity aqueous solution, wherein the low conductivity aqueous solution has a conductivity value of from 0.03 µS/cm to 0.5 mS/cm and wherein the low conductivity aqueous solution has a pH of about 7 or higher, and additionally washing the protein A affinity chromatography material with a medium conductivity aqueous solution before or after washing the protein A affinity chromatography material with a low conductivity aqueous solution, wherein the medium conductivity aqueous solution has a conductivity value of from more than 0.5 mS/cm to less than 20 mS/cm,
d) recovering the bispecific antibody from the protein A affinity chromatography material,
e) performing a further chromatography step
and thereby purifying the bispecific antibody.

In one embodiment of all aspects the further chromatography step after the protein A affinity chromatography step is an ion exchange chromatography step or a multimodal ion exchange chromatography step.

In one embodiment of all aspects the low conductivity aqueous solution comprises about 0.1 mM to about 8 mM Tris.

In one embodiment of all aspects the low conductivity aqueous solution comprises about 0.05 mM to about 2 mM potassium phosphate.

In one embodiment of all aspects the method additionally comprises washing the protein A affinity chromatography material with a high conductivity aqueous solution and with a medium conductivity aqueous solution before or after washing the protein A affinity chromatography material with a low conductivity aqueous solution, wherein the high conductivity aqueous solution has a conductivity value of about 20 mS/cm or higher and wherein the medium conductivity aqueous solution has a conductivity value of from more than 0.5 mS/cm to less than 20 mS/cm.

In one embodiment of all aspects the high or medium conductivity aqueous solution comprises Histidine.

In one embodiment of all aspects the bispecific antibody is an antibody of IgG1 or IgG4 isotype.

In one embodiment of all aspects the bispecific antibody is a bispecific antibody comprising
a) the heavy chain and the light chain of a first full length antibody that specifically binds to a first antigen;and
b) the heavy chain and the light chain of a second full length antibody that specifically binds to a second antigen, wherein the constant domains CL and CH1 are replaced by each other.

In one embodiment of all aspects the first antigen is human VEGF and the second antigen is human ANG-2 or the first antigen is human ANG-2 and the second antigen is human VEGF.

In one preferred embodiment of all aspects said first antigen-binding site comprises as heavy chain variable domain (VH) the SEQ ID NO: 1, and as light chain variable domain (VL) the SEQ ID NO: 2; and said second antigen-binding site comprises as heavy chain variable domain (VH) the SEQ ID NO: 3, and as light chain variable domain (VL) the SEQ ID NO: 4.

### Detailed Description of the Invention

In general, high conductivity buffers are described to be employed in wash steps of affinity chromatrography methods. Due to e.g. high salt concentrations, further process steps may be necessary in the transition from one to another chromatography/separation step. High conductivity values in eluates from one chromatography step may have unfavorable effects on the subsequent chromatography step.

Herein is reported an improved purification process using an affinity chromatography method comprising the washing of the affinity chromatography material with a low conductivity aqueous solution and with a medium conductivity aqueous solution, followed by an additional separation step.

Reported herein is a method for producing a bispecific antibody comprising a first antigen-binding site that specifically binds to a first antigen and a second antigen-binding site that specifically binds to a second antigen comprising the following steps
a) cultivating a cell comprising a nucleic acid encoding the bispecific antibody,
b) recovering the bispecific antibody from the cell or the cultivation medium,
c) contacting (a solution comprising) the bispecific antibody with an affinity chromatography material,
d) washing the affinity chromatography material with a low conductivity aqueous solution, while at least 90% of the bispecific antibody remains bound to the affinity chromatography material,
e) recovering the bispecific antibody from the affinity chromatography material,
f) performing a further chromatography step
and thereby producing the bispecific antibody.

Reported herein is a method for purifying a bispecific antibody comprising a first antigen-binding site that specifically binds to a first antigen and a second antigen-binding site that specifically binds to a second antigen from a sample comprising the steps of
a) providing a (buffered aqueous) sample comprising the bispecific antibody,
b) applying the sample comprising the bispecific antibody to an affinity chromatography material,
c) washing the affinity chromatography material with a low conductivity aqueous solution, while at least 90% of the bispecific antibody remains bound to the affinity chromatography material,
d) recovering the bispecific antibody from the affinity chromatography material
e) performing a further chromatography step
and thereby purifying the bispecific antibody .

Reported herein is a method for producing a bispecific antibody comprising a first antigen-binding site that specifically binds to a first antigen and a second antigen-binding site that specifically binds to a second antigen comprising the following steps
a) cultivating a cell comprising a nucleic acid encoding the bispecific antibody,
b) recovering the bispecific antibody from the cell or the cultivation medium,
c) contacting (a solution comprising) the bispecific antibody with an affinity chromatography material,
d) washing the affinity chromatography material with a low conductivity aqueous solution, while at least 90% of the bispecific antibody remains bound to the affinity chromatography material, wherein the low conductivity aqueous solution has a conductivity value of about 0.5 mS/cm or less,
e) recovering the bispecific antibody from the affinity chromatography material,
f) performing a further chromatography step
and thereby producing the bispecific antibody.

Reported herein is a method for purifying a bispecific antibody comprising a first antigen-binding site that specifically binds to a first antigen and a second antigen-binding site that specifically binds to a second antigen from a sample comprising the steps of
a) providing a (buffered aqueous) sample comprising the bispecific antibody,
b) applying the sample comprising the bispecific antibody to an affinity chromatography material,
c) washing the affinity chromatography material with a low conductivity aqueous solution, while at least 90% of the bispecific antibody remains bound to the affinity chromatography material, wherein the low conductivity aqueous solution has a conductivity value of about 0.5 mS/cm or less,
d) recovering the bispecific antibody from the affinity chromatography material
e) performing a further chromatography step
and thereby purifying the bispecific antibody .

In one embodiment of all aspects the further chromatography step after the affinity chromatography step is an ion exchange chromatography step or a multimodal ion exchange chromatography step. In one preferred embodiment of all aspects the further chromatography step after the affinity chromatography step is a multimodal anion exchange chromatography step. In one embodiment of all aspects the further chromatography step after the affinity chromatography step is a multimodal cation exchange chromatography step. In one embodiment of all aspects the further chromatography step after the affinity chromatography step is a multimodal anion exchange chromatography material selected from the group comprising CaptoAdhere ImpRes material (a multimodal anion exchange chromatography medium having a matrix of high-flow agarose, a multimodal strong anion exchanger as ligand, an average particle size of 36-44 µm and an ionic capacity of 0.08 to 0.11 mmol Cl-/mL medium).

Generally, certain additional process steps, such as pH adjustment, buffer exchange, dilution, diafiltration or conductivity adjustment, have to be performed in the transition from one column material to another. This is for example the case for a Protein A chromatography eluate to be applied on a following ion exchange chromatography material/resin. Here, the conductivity value of the load cannot exceed certain low levels, if proper functionality of the chromatography resins is needed. For example, removal of certain impurities can be negatively affected. It has been found that further process steps can be obviated before loading the eluate to the next column/chromatographic material, if a low conductivity aqueous solution wash step is used in the preceeding affinity chromatography step. This wash step is capable of having a significant influence on the final conductivity in the affinity chromatography (e.g. Protein A) eluate.

**Table 1**

| | Conductivity in Protein A eluate after conditioning [mS/cm] | Dilution necessary before loading 2^{nd} column (ion exchange) | Yield |
|---|---|---|---|
| Protein A chromatography without low conductivity wash | 6.0 | YES | 74.97 |
| Protein A chromatography with low conductivity wash | 3.8 | NO | 74.96 |

It has been found that the content of a host cell protein can be reduced if the conductivity of the aqueous solution used in the wash step is low i.e a low conductivity aqueous solution is used for washing. The low conductivity aqueous solution has a conductivity value of from about 0.03 µS/cm to about 0.5 mS/cm. In one embodiment the low conductivity aqueous solution has a conductivity value of from about 0.05 µS/cm to about 0.35 mS/cm. In one embodiment the low conductivity aqueous solution is highly purified/deionized water.

It has been found that a protein A affinity chromatography can be used for the purposes as reported herein. In one preferred embodiment of all aspects the affinity chromatography is a protein A affinity chromatography. In one embodiment the protein A affinity chromatography is selected from the group comprising MabSelectSure affinity chromatography, ProSep vA affinity chromatography, Mab Capture A affinity chromatography, ProSep Ultra Plus affinity chromatography . In one embodiment the affinity chromatography is a protein G affinity chromatography. In one embodiment the affinity chromatography is an affinity chromatography that uses a recombinant protein as a ligand, that means that the affinity chromatography is a recombinant protein ligand affinity chromatography. In one embodiment the affinity chromatography is an affinity chromatography that uses a single chain Fv as a ligand, that means that the affinity chromatography is a single chain Fv ligand affinity chromatography. In one embodiment the affinity chromatography comprises a mutated Protein A coupled to a chromatography matrix or a fragment of Protein A coupled to a chromatography matrix.

It has been found that the content of (specific) host cell proteins can be reduced. It has been found that especially the content of phospholipase B-like 2 (PLBL2) can be reduced. In one embodiment the (specific) host cell protein is a Chinese hamster ovary (CHO) host cell protein. In one preferred embodiment of all aspects the (specific) host cell protein is phospholipase B-like 2 (PLBL2) or Clusterin. In one embodiment the (specific) host cell protein is phospholipase B-like 2 (PLBL2).

It has been found that low conductivity aqueous solution may comprise Tris or potassium phosphate in low amounts. In one embodiment the low conductivity aqueous solution contains tris(hydroxymethyl)aminomethane (Tris). In one embodiment the low conductivity aqueous solution comprises about 0.1 mM to about 10 mM Tris. In one embodiment the low conductivity aqueous solution comprises about 0.5 mM to about 6.5 mM Tris. In one embodiment the low conductivity aqueous solution comprises about 2 mM Tris. In one embodiment the low conductivity aqueous solution contains potassium phosphate. In one embodiment the low conductivity aqueous solution comprises about 0.05 mM to about 5 mM potassium phosphate. In one embodiment the low conductivity aqueous solution comprises about 0.05 mM to about 2 mM potassium phosphate. In one embodiment the low conductivity aqueous solution comprises about 0.5 mM potassium phosphate.

It has been found that the effect of reducing the content of a host cell protein is pronounced if the low conductivity aqueous solution has a certain pH. In one embodiment the low conductivity aqueous solution has a pH of about 7 or higher. In one embodiment the low conductivity aqueous solution has a pH of about 7.5 or higher. In one embodiment the low conductivity aqueous solution has a pH of from about 7.5 to about 8.5. In one embodiment the low conductivity aqueous solution has a pH of about 8.

It has been found that the methods as reported herein the content of host cell proteins like PLBL2 can be reduced to a certain level, e.g. when compared to the load amount of PLBL2 prior to a purification step like an affinity chromatoghraphy step. In one embodiment the content of PLBL2 is reduced at least 20-fold. In one embodiment the content of PLBL2 is reduced at least 40-fold. In one embodiment the content of PLBL2 is reduced at least 50-fold. In one embodiment the content of PLBL2 is reduced at least 90-fold. In one embodiment the content of PLBL2 is reduced at least 100-fold. In one embodiment the content of PLBL2 is reduced at least by 50%. In one embodiment the content of PLBL2 is reduced at least by 66%. In one embodiment the content of PLBL2 is reduced at least by 80%. In one embodiment the content of PLBL2 is reduced at least by 90%. In one embodiment the content of PLBL2 is reduced at least by 95%. In some embodiments the content of PLBL2 is reduced to below 10 ng per mg of antibody. In some embodiments the content of PLBL2 is reduced to below 5 ng per mg of antibody. In some embodiments the content of PLBL2 is reduced to below 2 ng per mg of antibody.

In the methods as reported herein further wash steps can be employed with medium and/or high conductivity aqueous solutions. In one embodiment the low conductivity aqueous solution wash step is preceded or succeeded by a high conductivity aqueous solution wash step. In one embodiment the high conductivity aqueous solution has a conductivity value of about 20 mS/cm or higher. In one embodiment the high conductivity aqueous solution has a conductivity value of from about 20 mS/cm to about 100 mS/cm. In one embodiment an intermediate wash step is performed with a medium conductivity aqueous solution between the low conductivity aqueous solution wash step and the high conductivity aqueous solution wash step. The medium conductivity aqueous solution has a conductivity value of from more than 0.5mS/cm to less than 20 mS/cm.

It has been found that the host cell protein reducing effect can be improved when the high or medium conductivity aqueous solution further comprises an amino acid. In one embodiment the high or medium conductivity aqueous solution comprises an amino acid. In one embodiment the high or medium conductivity aqueous solution comprises Histidine. In one embodiment the high or medium conductivity aqueous solution comprises Histidine and Tris.

It has been found that the use of a hydrophobic interaction chromatography step may be omitted. In one embodiment the use or the methods is without an hydrophobic interaction chromatography method/step.

One aspect as reported herein is a method for producing a bispecific antibody comprising a first antigen-binding site that specifically binds to a first antigen and a second antigen-binding site that specifically binds to a second antigen comprising
a) cultivating a cell comprising a nucleic acid encoding the bispecific antibody,
b) recovering the bispecific antibody from the cell or the cultivation medium,
c) contacting the bispecific antibody with a Protein A affinity chromatography material,
d) washing the Protein A affinity chromatography material with a low conductivity aqueous solution, wherein the low conductivity aqueous solution has a conductivity value of from 0.03 µS/cm to 0.5 mS/cm, and additionally washing the affinity chromatography material with a medium conductivity aqueous solution before or after washing the affinity chromatography material with a low conductivity aqueous solution, wherein the medium conductivity aqueous solution has a conductivity value of from more than 0.5 mS/cm to less than 20 mS/cm,
e) recovering the bispecific antibody from the Protein A affinity chromatography material,
f) performing a further chromatography step

and thereby producing the bispecific antibody,
wherein the further chromatography step after the affinity chromatography step a multimodal anion exchange chromatography step, and wherein the low conductivity aqueous solution is water, and wherein said first antigen-binding site comprises as heavy chain variable domain (VH) the SEQ ID NO: 1, and as light chain variable domain (VL) the SEQ ID NO: 2; and said second antigen-binding site comprises as heavy chain variable domain (VH) the SEQ ID NO: 3, and as light chain variable domain (VL) the SEQ ID NO: 4.

One aspect as reported herein is a method for purifying a bispecific antibody comprising a first antigen-binding site that specifically binds to a first antigen and a second antigen-binding site that specifically binds to a second antigen from a sample comprising the steps of
a) providing a sample comprising the bispecific antibody,
b) applying the sample comprising the bispecific antibody to a Protein A affinity chromatography material,
c) washing the Protein A affinity chromatography material with a low conductivity aqueous solution, wherein the low conductivity aqueous solution has a conductivity value of from 0.03 µS/cm to 0.5 mS/cm, and additionally washing the affinity chromatography material with a medium conductivity aqueous solution before or after washing the affinity chromatography material with a low conductivity aqueous solution, wherein the medium conductivity aqueous solution has a conductivity value of from more than 0.5 mS/cm to less than 20 mS/cm,
d) recovering the bispecific antibody from the Protein A affinity chromatography material
e) performing a further chromatography step

and thereby purifying the bispecific antibody,
wherein the further chromatography step after the affinity chromatography step a multimodal anion exchange chromatography step, and wherein the low conductivity aqueous solution is water, and wherein said first antigen-binding site comprises as heavy chain variable domain (VH) the SEQ ID NO: 1, and as light chain variable domain (VL) the SEQ ID NO: 2; and said second antigen-binding site comprises as heavy chain variable domain (VH) the SEQ ID NO: 3, and as light chain variable domain (VL) the SEQ ID NO: 4.

One aspect as reported herein is a method for producing a bispecific antibody comprising a first antigen-binding site that specifically binds to a first antigen and a second antigen-binding site that specifically binds to a second antigen comprising the following steps
a) cultivating a cell comprising a nucleic acid encoding the bispecific antibody,
b) recovering the bispecific antibody from the cell or the cultivation medium,
c) contacting the bispecific antibody with an affinity chromatography material,
d) washing the affinity chromatography material with a low conductivity aqueous solution, wherein the low conductivity aqueous solution has a conductivity value of from 0.03 µS/cm to 0.5 mS/cm, and additionally washing the affinity chromatography material with a medium conductivity aqueous solution before or after washing the affinity chromatography material with a low conductivity aqueous solution, wherein the medium conductivity aqueous solution has a conductivity value of from more than 0.5 mS/cm to less than 20 mS/cm,,
e) recovering the bispecific antibody from the affinity chromatography material,
f) performing a further chromatography step
and thereby producing the bispecific antibody.

One aspect as reported herein is a method for purifying a bispecific antibody comprising a first antigen-binding site that specifically binds to a first antigen and a second antigen-binding site that specifically binds to a second antigen comprising the following steps
a) cultivating a cell comprising a nucleic acid encoding the bispecific antibody,
b) recovering the bispecific antibody from the cell or the cultivation medium,
c) contacting the bispecific antibody with an affinity chromatography material,
d) washing the affinity chromatography material with a low conductivity aqueous solution, wherein the low conductivity aqueous solution has a conductivity value of from 0.03 µS/cm to 0.5 mS/cm, and additionally washing the affinity chromatography material with a medium conductivity aqueous solution before or after washing the affinity chromatography material with a low conductivity aqueous solution, wherein the medium conductivity aqueous solution has a conductivity value of from more than 0.5 mS/cm to less than 20 mS/cm,,
e) recovering the bispecific antibody from the affinity chromatography material,
f) performing a further chromatography step
and thereby producing the bispecific antibody.

The term "method" as used herein also encompasses a use of the respective method steps for obviating a conductivity adjusting step prior to the (directly) following, further chromatography step. Especially, the obviating of the conductivity adjustment is after a viral inactivation step and before a (directly) thereafter performed ion exchange chromatography step.

Reported herein is the use of the steps of the methods as reported herein for obviating a conductivity adjusting step after recovering the bispecific antibody from the affinity chromatography material and prior to performing a further chromatography step.

The terms "anti-Ang2/VEGF antibody" and "a bispecific antibody that binds to Ang2 and VEGF" or "bispecific antibody against Ang2 and VEGF"refer to an antibody that is capable of binding Ang2 and VEGF with sufficient affinity such that the antibody is useful as a diagnostic and/or therapeutic agent in targeting Ang2 and VEGF. In one embodiment, the extent of binding of an anti-Ang2/VEGF antibody to an unrelated, non- Ang2 or VEGF protein is less than about 10% of the binding of the antibody to Ang2 and VEGFas measured, e.g., by ELISA or surface plasmon resonance. In certain embodiments, an anti- Ang2/VEGF antibody binds to an epitope of Ang2 and to an epitope of VEGF that is conserved among Ang2 or VEGF from different species. The above also holds for the terms "bispecific antibody against factor IXa and factor X" or "bispecific antibody against Her3 and EGFR" or the like.

The specific antibodies to be used in the methods as reported herein are a bispecific antibody against factor IXa and factor X (anti-FIXa/X antibody; IgG4 isotype) as described in WO 2012/067176, a bispecific antibody against angiopoietin 2 (Ang2) and vascular endothelial growth factor A (VEGF-A) (anti-Ang2/VEGF-A antibody; vanucizumab; IgG1 isotype) as described in WO 2011/117329 or SEQ ID NO: 01 to 04, or a bispecific antibody against Her3 and EGFR (anti-Her3/EGFR antibody; IgG1 isotype). The terms VEGF or VEGF-A can be used interchangeably herein.

As used herein, the term "binding" or "specifically binding" refers to the binding of the antibody to an epitope of the antigen in an in-vitro assay, preferably in a surface plasmon resonance assay (SPR, BIAcore, GE-Healthcare Uppsala, Sweden). The affinity of the binding is defined by the terms ka (rate constant for the association of the antibody from the antibody/antigen complex), k_{d} (dissociation constant), and K_{D} (k_{d}/kₐ). Binding or specifically binding means a binding affinity (K_{D}) of 10⁻⁷ mol/L or less.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g. scFv); and multispecific antibodies formed from antibody fragments. A Fab fragement is an antibody fragment obtained by a papain digestion of a (full length/complete) antibody.

Bispecific antibodies" are antibodies which have two different antigen-binding specificities. The term "bispecific" antibody as used herein denotes an antibody that has at least two binding sites each of which bind to different epitopes.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively.

The term "Fc-region" herein is used to define a C-terminal region of an immunoglobulin heavy chain that contains at least a portion of the constant region. The term includes native sequence Fc-regions and variant Fc-regions. In one embodiment, a human IgG heavy chain Fc-region extends from Cys226, or from Pro230, to the carboxyl-terminus of the heavy chain. However, the C-terminal lysine (Lys447) or the C-terminal glycyl-lysine dipeptide (Gly446Lys447) of the Fc-region may or may not be present. Unless otherwise specified herein, numbering of amino acid residues in the Fc-region or constant region is according to the EU numbering system, also called the EU index, as described in Kabat, E.A. et al., Sequences of Proteins of Immunological Interest, 5th ed., Public Health Service, National Institutes of Health, Bethesda, MD (1991), NIH Publication 91-3242.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "host cell", "host cell line", and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. The term "cell" includes cells which are used for the expression of nucleic acids. In one embodiment the host cell is a CHO cell (e.g. CHO K1, CHO DG44), or a BHK cell, or a NS0 cell, or a SP2/0 cell, or a HEK 293 cell, or a HEK 293 EBNA cell, or a PER.C6^{®} cell, or a COS cells. In another embodiment the cell is a CHO cell, or a BHK cell, or a PER.C6^{®} cell. As used herein, the expression "cell" includes the subject cell and its progeny.

The term "washing" denotes the applying of a solution to an affinity chromatography material in order to remove non specifically bound polypeptides and non-polypeptide compounds from the chromatography material, especially to remove host cell protein and host cell DNA. The term "washing" does not encompass the elution of bound material from an affinity chromatography material.

Different methods are well established and widespread used for protein recovery and purification, such as affinity chromatography with microbial proteins (e.g. protein A or protein G affinity chromatography) affinity chromatographie with a recombinant protein as ligand (e.g. single chain Fv as ligand, e.g. Kappa select), ion exchange chromatography (e.g. cation exchange (carboxymethyl resins), anion exchange (amino ethyl resins) and mixed-mode exchange), thiophilic adsorption (e.g. with beta-mercaptoethanol and other SH ligands), hydrophobic interaction or aromatic adsorption chromatography (e.g. with phenyl-sepharose, aza-arenophilic resins, or m-aminophenylboronic acid), metal chelate affinity chromatography (e.g. with Ni(II)- and Cu(II)-affinity material), size exclusion chromatography, and electrophoretical methods (such as gel electrophoresis, capillary electrophoresis). These methods can be combined independently in different embodiments as reported herein.

The term "protein A" denotes a protein A polypeptide either obtained from a natural source or produced synthetically.

The term "protein A chromatography material" denotes an inert solid phase to which a protein A is covalently linked.

In one embodiment the protein A chromatography material is selected from MabSelectSure, ProSep vA, Mab Capture A, ProSep Ultra Plus, Mab Select, Mab Select Xtra, Poros A, or ProSep A.

The term "high conductivity aquaeous solution"denotes an aquaeous solution with a high conductivity value. The conductivity value is 20 mS/cm or higher.

The term "medium conductivity aquaeous solution"denotes an aquaeous solution with a medium conductivity value. The conductivity value is from more than 0.5 mS/cm to less than 20 mS/cm.

The term "low conductivity aquaeous solution"denotes an aquaeous solution with a low conductivity value. The conductivity value is from 0.03 µS/cm to 0.5 mS/cm The following examples and sequences are provided to aid the understanding of the present invention, the true scope of which is set forth in the appended claims.

### Description of the Sequence Listing

| | |
|---|---|
| **SEQ ID NO: 01** | variable heavy chain domain VH of <VEGF> |
| **SEQ ID NO: 02** | variable light chain domain VL of <VEGF> |
| **SEQ ID NO: 03** | variable heavy chain domain VH of <ANG-2> |
| **SEQ ID NO: 04** | variable light chain domain VL of < ANG-2> |

### Example 1

### Material and Methods

### Antibodies

The current invention is exemplified with a bispecific antibody against factor IXa and factor X (anti-FIXa/X antibody; IgG4 isotype) as described in WO 2012/067176, with a bispecific antibody against Ang2 and VEGF-A (anti-Ang2/VEGF-A antibody; vanucizumab; IgG1 isotype) as described in WO 2011/117329 or SEQ ID NO: 01 to 04, or with a bispecific antibody against Her3 and EGFR (anti-Her3/EGFR antibody; IgG1 isotype).

### Detection methods for overall host cell protein (HCP), Phospholipase B-like 2 protein (PLBL2) and Clusterin

### a) CHO HCP assay

The residual CHO HCP content in process samples is determined by an electrochemiluminescence immunoassay (ECLIA) on cobas e 411 immunoassay analyzer (Roche Diagnostics).

The assay is based on a sandwich principle using polyclonal anti-CHO HCP antibody from sheep.

First incubation: Chinese hamster ovary host cell protein (CHO HCP) from 15 µL sample (neat and/or diluted) and a biotin conjugated polyclonal CHO HCP specific antibody form a sandwich complex, which becomes bound to streptavidin-coated microparticles via interaction of biotin with streptavidin.

Second incubation: After addition of polyclonal CHO HCP-specific antibody labeled with ruthenium complex (Tris(2,2'-bipyridyl)ruthenium(II)-complex) a ternary sandwich complex is formed on the microparticles.

The reaction mixture is aspirated into the measuring cell where the microparticles are magnetically captured onto the surface of the electrode. Unbound substances are then removed in a washing step. Application of a voltage to the electrode then induces chemiluminescent emission which is measured by a photomultiplier.

The concentration of CHO HCP in the test sample is finally calculated from a CHO HCP standard curve of known concentration.

### b) CHO PLBL2 assay

The residual Chinese hamster ovary (CHO) Phospholipase B-like 2 protein (PLBL2) content in process samples is determined by an electrochemiluminescence immunoassay (ECLIA) on cobas e 411 immunoassay analyzer (Roche Diagnostics).

The assay is based on a sandwich principle using monoclonal anti-CHO PLBL2 antibody from mouse.

In a first incubation step, CHO PLBL2 from 30 µL sample (neat and/or diluted), biotin labeled monoclonal CHO PLBL2-specific antibody, and a monoclonal CHO PLBL2-specific antibody labeled with a ruthenium complex (Tris(2,2'-bipyridyl)ruthenium(II)-complex) form a sandwich complex.

In a second step after addition of streptavidin-coated microparticles, the ternary complex becomes bound to the solid phase via interaction of biotin and streptavidin.

The reaction mixture is aspirated into the measuring cell where the microparticles are magnetically captured onto the surface of the electrode. Unbound substances are then removed in a washing step. Application of a voltage to the electrode then induces chemiluminescence, which is measured by a photomultiplier.

The concentration of CHO PLBL2 in the test sample is finally calculated from a CHO PLBL2 standard curve of known concentration.

### c) Clusterin assay

The residual Clusterin content in process samples is determined by a commercial assay from Merck Millipore (GyroMark HT Kit GYRCLU-37K) which was used according to the manufacturer's instructions.

In brief, this assay is a Sandwich ELISA based, sequentially, on:
1) binding of the rat Clusterin biotinylated capture antibody to the streptavidin coated affinity columns of the Bioaffy 1000nL CD,
2) capture of rat Clusterin molecules from samples to the anti Clusterin antibody,
3) binding of a second dye-labeled anti Clusterin detection antibody to the captured molecules,
4) quantification of the rat Clusterin using the Gyrolab Evaluator.

### Example 2

### Purification of a bispecific anti-Ang2/VEGF-A antibody (IgG1 isotype) in a protein A chromatography

Antibody: anti-Ang2/VEGF-A antibody

### General chromatography conditions:

Column resin: Protein A material "Mab Select SuRe" (GE-Healthcare) Ø 1cm,
Height: 20,1 cm, CV: 15,79 ml
Equipment: Äkta Avant 150
Flow rate: 300 cm/h during all steps

A solution containing an anti-Ang2/VEGF-A antibody, was applied to a Protein A affinity column after equilibration (step 1) of the column. Initial load of PLBL2 determined in solution containing an anti-Ang2/VEGF-A antibody: 919.7 ng PLBL2/mg of antibody. Initial load of CHOP determined in solution containing an anti-Ang2/VEGF-A antibody: 682304 ng CHOP/mg of antibody.

The chromatographic steps were performed according to the following general scheme:
Step 1: Equilibration:
Step 2: Load of antibody containing solution
Step 3: Wash I
Step 4: Wash II
Step 5: Wash III
Step 6: Wash IV (additional wash)
Step 7: Elution

After Elution from Protein A affinity column the protein was determined by size exclusion chromatography (SEC) and spectrophotometrically (OD) Analytics.

### SEC:

| | |
|---|---|
| Resin: | TSK 3000 (Tosoh) |
| Column: | 300 x 7,8 mm |
| Flow rate: | 0,5 ml/min |
| Buffer: | 200 mM potassium phosphate containing |
| | 250 mM potassium chloride, adjusted to pH 7,0 |
| Wavelength: | 280 nm |

### OD:

| | |
|---|---|
| Specific coefficient: | 1,54 |
| Wavelength: | 280 nm minus 320 nm |

Specific buffer conditions for Protein A chromatography
a) Control (wash with equilibration buffer only)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 25 mM Tris, 25 mM NaCl, pH 7,0 |
| Step 5: Wash III: | --- |
| *Step 6: Wash IV:* | --- |
| Step 7: Elution: | 50 mM acedic acid, pH 4,0 |

b) low conductivity wash (with Tris buffer only)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 25 mM Tris, 25 mM NaCl, pH 7,0 |
| Step 5: Wash III: | 25 mM Tris, 25 mM NaCl, pH 7,0 |
| *Step 6: Wash IV:* | 2 mM Tris, pH 8.0 |
| Step 7: Elution: | 50 mM acedic acid, pH 4,0 |

c) high conductivity wash (with Tris buffer only)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 700 mM Tris, pH 7,2 |
| Step 5: Wash III: | 25 mM Tris, 25 mM NaCl, pH 7,0 |
| *Step 6: Wash IV:* | --- |
| Step 7: Elution: | 50 mM acedic acid, pH 4,0 |

d) low conductivity Tris + high conductivity Histidine (His)/Tris

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 200 mM His/1000 mM Tris, pH 7,0 |
| Step 5: Wash III: | 25 mM Tris, 25 mM NaCl, pH 7,0 |
| *Step 6: Wash IV:* | 2 mM Tris, pH 8.0 |
| Step 7: Elution: | 50 mM acedic acid, pH 4,0 |

| Run | HCP total [ng/mg] | PLBL2 [ng/mg] | Clusterin [ng/mg] | Yield [%] |
|---|---|---|---|---|
| a | 3035 | 1.0 | n.d. | 85.0 |
| b | 1707 | 0.8 | n.d. | 79.8 |
| c | 655 | 0.7 | n.d. | 52 |
| d | 1050 | 0.8 | n.d. | 92.3 |

### Example 3

### Purification of a bispecific anti-FIXa/X antibody (IgG4 isotype) in a protein A chromatography

Purification of anti-FIXa/X antibody was tested in two different chromatograhpy settings:

### Setting 1

General conditions were according to the conditions described in Example 2.
Antibody: anti-FIXa/X

Initial load of PLBL2 determined in solution containing an anti-FIXa/X antibody: 557 ng PLBL2/mg of antibody. Initial load of CHOP determined in solution containing an anti-FIXa/X: 387377 ng CHOP/mg of antibody.

Specific buffer conditions for Protein A chromatography
a) high conductivity wash (with Tris buffer only)

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 700 mM Tris, pH 7,2 |
| Step 5: Wash III: | 25 mM Tris, 25 mM NaCl, pH 7,0 |
| *Step 6: Wash IV:* | --- |
| Step 7: Elution: | 50 mM acedic acid, pH 4,0 |

b) low conductivity Tris + high conductivity Histidine (His)/Tris

| | |
|---|---|
| Step 1: Equilibration: | 25 mM Tris, 25 mM NaCl, pH 7,0 |
| Step 2: Load | |
| Step 3: Wash I: | 25 mM Tris, 25 mM NaCl, pH 7,0 |
| Step 4: Wash II: | 200 mM His/1000 mM Tris, pH 7,0 |
| Step 5: Wash III: | 25 mM Tris, 25 mM NaCl, pH 7,0 |
| *Step 6: Wash IV:* | 2 mM Tris, pH 8.0 |
| Step 7: Elution: | 50 mM acedic acid, pH 4,0 |

| Run | HCP total [ng/mg] | PLBL2 [ng/mg] | Clusterin [ng/mg] | Yield [%] |
|---|---|---|---|---|
| a | 1632 | 19.1 | n.d. | 79 |
| b | 2148 | 1.1 | n.d. | 77 |

### Setting 2

### General chromatography conditions

Column resin: Protein A material "Mab Select SuRe" (GE-Healthcare) Ø 1cm,
Height: 20,1 cm, CV: 15,79 ml
Equipment: Äkta Avant 150
Flow rate: 300 cm/h during all steps

A solution containing an anti-FIXa/X antibody, was applied to a Protein A affinity column after equilibration (step 1) of the column.

Initial load of PLBL2 determined in solution containing an anti-FIXa/X antibody: 557 ng PLBL2/mg of antibody. Initial load of CHOP determined in solution containing an anti-FIXa/X: 387377 ng CHOP/mg of antibody.

The chromatographic steps were performed according to the following general scheme:
Step 1: Equilibration:
Step 2: Load of antibody containing solution
Step 3: Wash I
Step 4: Wash II
Step 5: Wash III (additional wash)
Step 6: Elution

Specific buffer conditions for Protein A chromatography
a) high conductivity wash (with NaSO4 buffer only)

| | |
|---|---|
| Step 1: Equilibration: | 20 mM NaPO4, pH 7,5 |
| Step 2: Load | |
| Step 3: Wash I: | 450 mM NaSO4, 20 mM NaAc, pH 4,8 |
| Step 4: Wash II: | 20 mM NaPO4, pH 7,5 |
| *Step 5: Wash III:* | --- |
| Step 6: Elution: | 35 mM acedic acid, pH 4,0 |

b) low conductivity wash (Tris 1 mM) + high conductivity wash (with NaSO4)

| | |
|---|---|
| Step 1: Equilibration: | 20 mM NaPO4, pH 7,5 |
| Step 2: Load | |
| Step 3: Wash I: | 450 mM NaSO4, 20 mM NaAc, pH 4,8 |
| Step 4: Wash II: | 20 mM NaPO4, pH 7,5 |
| *Step 5: Wash III:* | 1 mM Tris, pH 8.0 |
| Step 6: Elution: | 50 mM acedic acid, pH 4,0 |

c) low conductivity wash (Tris 2 mM) + high conductivity wash (with NaSO4)

| | |
|---|---|
| Step 1: Equilibration: | 20 mM NaPO4, pH 7,5 |
| Step 2: Load | |
| Step 3: Wash I: | 450 mM NaSO4, 20 mM NaAc, pH 4,8 |
| Step 4: Wash II: | 20 mM NaPO4, pH 7,5 |
| *Step 5: Wash III:* | 2 mM Tris, pH 8.0 |
| Step 6: Elution: | 35 mM acedic acid, pH 4,0 |

d) low conductivity wash (Tris 4 mM) + high conductivity wash (with NaSO4)

| | |
|---|---|
| Step 1: Equilibration: | 20 mM NaPO4, pH 7,5 |
| Step 2: Load | |
| Step 3: Wash I: | 450 mM NaSO4, 20 mM NaAc, pH 4,8 |
| Step 4: Wash II: | 20 mM NaPO4, pH 7,5 |
| *Step 5: Wash III:* | 4 mM Tris, pH 8.0 |
| Step 6: Elution: | 50 mM acedic acid, pH 4,0 |

e) low conductivity wash (Tris 6 mM) + high conductivity wash (with NaSO4)

| | |
|---|---|
| Step 1: Equilibration: | 20 mM NaPO4, pH 7,5 |
| Step 2: Load | |
| Step 3: Wash I: | 450 mM NaSO4, 20 mM NaAc, pH 4,8 |
| Step 4: Wash II: | 20 mM NaPO4, pH 7,5 |
| *Step 5: Wash III:* | 6 mM Tris, pH 8.0 |
| Step 6: Elution: | 50 mM acedic acid, pH 4,0 |

f) low conductivity wash (Tris 4 mM, pH 7.8) + high conductivity wash (with NaSO4)

| | |
|---|---|
| Step 1: Equilibration: | 20 mM NaPO4, pH 7,5 |
| Step 2: Load | |
| Step 3: Wash I: | 450 mM NaSO4, 20 mM NaAc, pH 4,8 |
| Step 4: Wash II: | 20 mM NaPO4, pH 7,5 |
| *Step 5: Wash III:* | 4 mM Tris, pH 7.8 |
| Step 6: Elution: | 50 mM acedic acid, pH 4,0 |

g) low conductivity wash (Tris 4 mM, pH 8.2) + high conductivity wash (with NaSO4)

| | |
|---|---|
| Step 1: Equilibration: | 20 mM NaPO4, pH 7,5 |
| Step 2: Load | |
| Step 3: Wash I: | 450 mM NaSO4, 20 mM NaAc, pH 4,8 |
| Step 4: Wash II: | 20 mM NaPO4, pH 7,5 |
| *Step 5: Wash III:* | 4 mM Tris, pH 8.2 |
| Step 6: Elution: | 50 mM acedic acid, pH 4,0 |

h) low conductivity wash (Tris 2 mM) + high conductivity wash ( with Histidine (His)/Tris 1 M)

| | |
|---|---|
| Step 1: Equilibration: | 20 mM NaPO4, pH 7,5 |
| Step 2: Load | |
| Step 3: Wash I: | 200 mM His/1000 mM Tris, pH 7,0 |
| Step 4: Wash II: | 20 mM NaPO4, pH 7,5 |
| *Step 5: Wash III:* | 2 mM Tris, pH 8.0 |
| Step 6: Elution: | 35 mM acedic acid, pH 4,0 |

i) low conductivity wash (Tris 2 mM) + high conductivity wash (Histidine (His)/Tris 0.85 M)

| | |
|---|---|
| Step 1: Equilibration: | 20 mM NaPO4, pH 7,5 |
| Step 2: Load | |
| Step 3: Wash I: | 200 mM His/850 mM Tris, pH 7,0 |
| Step 4: Wash II: | 20 mM NaPO4, pH 7,5 |
| *Step 5: Wash III:* | 2 mM Tris, pH 8.0 |
| Step 6: Elution: | 50 mM acedic acid, pH 4,0 |

j) low conductivity wash (Tris 2 mM) + high conductivity wash (Histidine (His)/Tris 0.7 M)

| | |
|---|---|
| Step 1: Equilibration: | 20 mM NaPO4, pH 7,5 |
| Step 2: Load | |
| Step 3: Wash I: | 200 mM His/700 mM Tris, pH 7,0 |
| Step 4: Wash II: | 20 mM NaPO4, pH 7,5 |
| *Step 5: Wash III:* | 2 mM Tris, pH 8.0 |
| Step 6: Elution: | 50 mM acedic acid, pH 4,0 |

k) low conductivity wash (Tris 2 mM) + high conductivity wash (Histidine (His)/Tris 0.55 M)

| | |
|---|---|
| Step 1: Equilibration: | 20 mM NaPO4, pH 7,5 |
| Step 2: Load | |
| Step 3: Wash I: | 200 mM His/550 mM Tris, pH 7,0 |
| Step 4: Wash II: | 20 mM NaPO4, pH 7,5 |
| *Step 5: Wash III:* | 2 mM Tris, pH 8.0 |
| Step 6: Elution: | 50 mM acedic acid, pH 4,0 |

| Run | HCP total [ng/mg] | PLBL2 [ng/mg] | Clusterin [ng/mg] | Yield [%] |
|---|---|---|---|---|
| a | 1518 | 204.2 | n.d. | 82 |
| b | 646 | 1 | n.d. | 73.8 |
| c | 737 | 1.2 | n.d. | 79 |
| d | 595 | 1.4 | n.d. | 78.5 |
| e | 685 | 1.8 | n.d. | 79.5 |
| f | 692 | 1.4 | n.d. | 78.2 |
| g | 707 | 1.1 | n.d. | 76.4 |
| h | 299 | 0.5 | n.d. | 79 |
| i | 140 | 0.4 | n.d. | 70 |
| j | 100 | 0.5 | n.d. | 71.9 |
| k | 112 | 0.7 | n.d. | 73 |

### Example 4

### General procedure/conditions:

### Mock Cell Culture Fluid

Null harvested cell culture fluid was produced using non-transfected CHO-DP12 cells cultured in serum-free media. Fermentation was performed at the 2 L-scale using a representative cell culture process. At the end of 14 days of fermentation, cell culture fluid was harvested via centrifugation and sterile filtration. This harvested cell culture fluid (HCCF) was then stored at -70 °C until experimentation.

### Purified PLBL2

Recombinant CHO PLBL2 with a C-terminal hexahistidine-tag was expressed in 35 L-scale transient transfections and purified from harvested cell culture fluid as previously described (Vanderlaan et al., 2015). Purified PLBL2 was then formulated in a PBS solution and stored at -70°C until experimentation.

### Purified Antibody

Recombinant humanized antibodies (a bispecific antibody against Her3 and EGFR (anti-Her3/EGFR antibody; IgG1 isotype)) were expressed in CHO cells and purified using column chromatography to ensure PLBL2 concentration was below 20 ng/mg. Prior to beginning each study, each antibody was buffer-exchanged into PBS using PD-10 desalting columns (GE Healthcare).

### Preparation of Load Material for Protein A Chromatography

For packed-bed column chromatography, to normalize the population and abundance of host cell proteins in the Protein A load across antibodies, purified antibodies were diluted to the same concentration with PBS and spiked into HCCF from a non-producing cell line to give a final antibody titer of 5 g/L. A control was also prepared wherein PBS was added instead of the purified antibody to evaluate non-specific host cell protein binding to the Protein A resin in the absence of antibody.

For each high-throughput Protein A batch purification experiment, the load was prepared in 96-well plates. In each well, purified antibody was mixed with different ratios of PLBL2 and PBS to achieve the target relative PLBL2 concentrations: 0 - 20,000 ng/mg, and an antibody titer of 5 g/L.

### Packed-Bed Column Chromatography

All packed-bed column chromatography experiments were performed using a 0.66 cm inner diameter by 20 cm bed height MabSelect SuRe (GE Healthcare) Protein A resin column. For each purification, the column was first equilibrated for 3 column volumes (CVs) with 25 mM tris, 25 mM NaCl, pH 7.7 (equilibration buffer). Then Protein A load was applied to a target load density of 30 g antibody/Lresin, after which the column was washed for 3 CVs with equilibration buffer, 3 CVs of different types of washing buffers, and again with 3 CVs of equilibration buffer.

Subsequently, antibody was eluted at low pH with 0.1 M acetic acid, pH 2.8, and eluate pools were collected starting at 0.5 OD at the beginning of the elution peak; pooling was terminated after 2.8 CVs. For the control run with PBS-spiked null HCCF, a 2.8 CV mock elution pool was generated starting from 1 CV to 3.8 CVs after the start of the elution phase. At the end of every run, each Protein A eluate was then titrated to pH 5.0 using 1.5 M tris base. The column was then cleaned with a 0.1 M sodium hydroxide solution. All phases had a volumetric flow rate of 20 CV/h except for the load, first equilibration wash, and elution phases, which had a flow rate of 15 CV/h.

### A) Purification of an anti-Her3/EGFR antibody (IgG1 isotype) in a protein A chromatography

Specific washing buffer conditions for purification of an anti-Her3/EGFR antibody (IgG1 isotype) using the general procedure of Example 4:
a) Deionized water

| | HCP total [ng/mg] | PLBL2 [ng/mg] |
|---|---|---|
| | Load: 1067817 | Load: 7668 |
| a | 6427 | 28.36 |

### Example 5

### Purification of a bispecific anti-Ang2/VEGF-A antibody in a protein A chromatography with additional washing with highpure deionized water

Harvested cell culture fluid (HCCF) from a CHO expression culture was processed by MabSelect SuRe affinity chromatography in bind-elute mode. After loading of the HCCF onto the column to a maximum load density of 38 g_{mAb}/lᵣₑₛᵢₙ, the column was washed with 25 mM Tris, 25 mM NaCl, pH 7.2 for 5 column volumes. Then, an additional wash with 0.7 M Tris/HCl, pH 7.2 for five column volumes was performed. The third wash step was conducted using highly purified water for five column volumes. The column-bound antibody was eluted using 50 mM Acetate, pH 3.4. The elution pool was collected based on OD₂₈₀ from 500 to 250 mAU (path length 1cm), over a maximum of three column volumes.

The affinity elution pool was adjusted to pH 3.5 with acetic acid and held for 30min. The pool was then conditioned with 1.5 M Tris Base to pH 5.0 and cleared by depth filtration. The depth filtration pool was conditioned to pH 7.0 using 1.5 M Tris Base. The conductivity value of the conditioned pool was determined to be below 6 mS/cm. No dilution step with water is necessary before loading the material onto the next chromatography column, i.e. CaptoAdhere ImpRes.

### Results:

| Conductivity in Protein A eluate after conditioning [mS/cm] | Dilution (conductivity adjustment) necessary before loading 2^{nd} column (ion exchange) | HCP [ng/mg] | PLBL2 [ng/mg] | Yield |
|---|---|---|---|---|
| | | Load: 650717 ng/mg | Load: 584.6 ng/mg | |
| 3.8 | NO | 4124 | 1.2 | 74.96 |

### Example 6

### Purification of a bispecific anti-Ang2/VEGF-A antibody in a protein A chromatography without additional washing with highpure deionized water (comparative example)

Harvested cell culture fluid (HCCF) from a CHO expression culture was processed by MabSelect SuRe affinity chromatography in bind-elute mode. After loading of the HCCF onto the column to a maximum load density of 38 g_{mAb}/lᵣₑₛᵢₙ, the column was washed with 25 mM Tris, 25 mM NaCl, pH 7.2 for 5 column volumes. Then, an additional wash with 0.7 M Tris/HCl, pH 7.2 for five column volumes was performed. The third wash step was conducted using 25 mM Tris, 25 mM NaCl, pH 7.2 again. The column-bound antibody was eluted using 50 mM Acetate, pH 3.4. The elution pool was collected based on OD₂₈₀ from 500 to 250 mAU (path length 1cm), over a maximum of three column volumes.

The affinity elution pool was adjusted to pH 3.5 with acetic acid and held for 30min. The pool was then conditioned with 1.5 M Tris Base to pH 5.0 and cleared by depth filtration. The depth filtration pool was conditioned to pH 7.0 using 1.5 M Tris Base.

The conductivity value of the conditioned pool was determined to be at 6 mS/cm. An additional dilution step with water is necessary before loading the material onto the next chromatography column, i.e. CaptoAdhere ImpRes.

### Results:

| Conductivity in Protein A eluate after conditioning [mS/cm] | Dilution (conductivity adjustment) necessary before loading 2^{nd} column (ion exchange) | HCP [ng/mg] | PLBL2 [ng/mg] | Yield |
|---|---|---|---|---|
| | | Load: 650717 ng/mg | Load: 584.6 ng/mg | |
| 6.0 | YES | 3756 | 1.2 | 74.97 |

## Claims

1. Method for producing a bispecific antibody comprising a first antigen-binding site that specifically binds to a first antigen and a second antigen-binding site that specifically binds to a second antigen comprising the following steps
a) cultivating a cell comprising a nucleic acid encoding the bispecific antibody,
b) recovering the bispecific antibody from the cell or the cultivation medium,
c) contacting the bispecific antibody with a protein A affinity chromatography material,
d) washing the protein A affinity chromatography material with a low conductivity aqueous solution, wherein the low conductivity aqueous solution has a conductivity value of from 0.03 µS/cm to 0.5 mS/cm and wherein the low conductivity aqueous solution has a pH of about 7 or higher, and additionally washing the protein A affinity chromatography material with a medium conductivity aqueous solution before or after washing the protein A affinity chromatography material with a low conductivity aqueous solution, wherein the medium conductivity aqueous solution has a conductivity value of from more than 0.5 mS/cm to less than 20 mS/cm,
e) recovering the bispecific antibody from the protein A affinity chromatography material,
f) performing a further chromatography step,
and thereby producing the bispecific antibody.

2. Method for purifying a bispecific antibody comprising a first antigen-binding site that specifically binds to a first antigen and a second antigen-binding site that specifically binds to a second antigen from a sample comprising the steps of
a) providing a sample comprising the bispecific antibody,
b) applying the sample comprising the bispecific antibody to an protein A affinity chromatography material,
c) washing the protein A affinity chromatography material with a low conductivity aqueous solution, wherein the low conductivity aqueous solution has a conductivity value of from 0.03 µS/cm to 0.5 mS/cm and wherein the low conductivity aqueous solution has a pH of about 7 or higher, and additionally washing the protein A affinity chromatography material with a medium conductivity aqueous solution before or after washing the protein A affinity chromatography material with a low conductivity aqueous solution, wherein the medium conductivity aqueous solution has a conductivity value of from more than 0.5 mS/cm to less than 20 mS/cm,
d) recovering the bispecific antibody from the protein A affinity chromatography material
e) performing a further chromatography step,
and thereby purifying the bispecific antibody.

3. Method according to any one of claims 1 to 2, wherein the further chromatography step after the protein A affinity chromatography step is an ion exchange chromatography step or a multimodal ion exchange chromatography step.

4. Method according to any one of claims 1 to 3, wherein the low conductivity aqueous solution comprises about 0.1 mM to about 8 mM Tris.

5. Method according to any one of claims 1 to 3, wherein the low conductivity aqueous solution comprises about 0.05 mM to about 2 mM potassium phosphate.

6. Method according to any one of claims 1 to 5, wherein the method additionally comprises washing the protein A affinity chromatography material with a high conductivity aqueous solution and with a medium conductivity aqueous solution before or after washing the protein A affinity chromatography material with a low conductivity aqueous solution, wherein the high conductivity aqueous solution has a conductivity value of about 20 mS/cm or higher and wherein the medium conductivity aqueous solution has a conductivity value of from more than 0.5 mS/cm to less than 20 mS/cm.

7. Method according to claim 6, wherein the high or medium conductivity aqueous solution comprises Histidine.

8. Method according to any one of claims 1 to 7, wherein the bispecific antibody is an antibody of IgG1 or IgG4 isotype.

9. Method according to any one of claims 1 to 8, wherein the bispecific antibody is a bispecific antibody comprising a) the heavy chain and the light chain of a first full length antibody that specifically binds to a first antigen;and b) the heavy chain and the light chain of a second full length antibody that specifically binds to a second antigen, wherein the constant domains CL and CH1 are replaced by each other.

10. Method according to any one of claims 1 to 9, wherein the first antigen is human VEGF and the second antigen is human ANG-2 or the first antigen is human ANG-2 and the second antigen is human VEGF.

11. Method according to any one of claims 1 to 10, wherein said first antigen-binding site comprises as heavy chain variable domain (VH) the SEQ ID NO: 1, and as light chain variable domain (VL) the SEQ ID NO: 2; and said second antigen-binding site comprises as heavy chain variable domain (VH) the SEQ ID NO: 3, and as light chain variable domain (VL) the SEQ ID NO: 4.

## Patentansprüche

1. Verfahren zur Herstellung eines bispezifischen Antikörpers, umfassend eine erste Antigenbindungsstelle, die spezifisch an ein erstes Antigen bindet, und eine zweite Antigenbindungsstelle, die spezifisch an ein zweites Antigen bindet, umfassend die folgenden Schritte:
a) Kultivieren einer Zelle, umfassend eine Nukleinsäure, die für den bispezifischen Antikörper kodiert,
b) Gewinnen des bispezifischen Antikörpers aus der Zelle oder dem Kulturmedium,
c) Inkontaktbringen des bispezifischen Antikörpers mit einem Protein-A-Affinitätschromatographiematerial,
d) Waschen des Protein-A-Affinitätschromatographiematerials mit einer wässrigen Lösung mit niedriger Leitfähigkeit, wobei die wässrige Lösung mit niedriger Leitfähigkeit einen Leitfähigkeitswert von 0,03 µS/cm bis 0,5 mS/cm aufweist und wobei die wässrige Lösung mit niedriger Leitfähigkeit einen pH-Wert von etwa 7 oder höher aufweist, und zusätzliches Waschen des Protein-A-Affinitätschromatographiematerials mit einer wässrigen Lösung mit mittlerer Leitfähigkeit vor oder nach dem Waschen des Protein-A-Affinitätschromatographiematerials mit einer wässrigen Lösung mit niedriger Leitfähigkeit, wobei die wässrige Lösung mit mittlerer Leitfähigkeit einen Leitfähigkeitswert von mehr als 0,5 mS/cm bis weniger als 20 mS/cm aufweist,
e) Gewinnen des bispezifischen Antikörpers aus dem Protein-A-Affinitätschromatographiematerial,
f) Durchführen eines weiteren Chromatographieschritts,
und dadurch Herstellen des bispezifischen Antikörpers.

2. Verfahren zur Reinigung eines bispezifischen Antikörpers, umfassend eine erste Antigenbindungsstelle, die spezifisch an ein erstes Antigen bindet, und eine zweite Antigenbindungsstelle, die spezifisch an ein zweites Antigen bindet, aus einer Probe, umfassend die folgenden Schritte:
a) Bereitstellen einer Probe, umfassend den bispezifischen Antikörper,
b) Aufbringen der Probe, umfassend den bispezifischen Antikörper, auf ein Protein-A-Affinitätschromatographiematerial,
c) Waschen des Protein-A-Affinitätschromatographiematerials mit einer wässrigen Lösung mit niedriger Leitfähigkeit, wobei die wässrige Lösung mit niedriger Leitfähigkeit einen Leitfähigkeitswert von 0,03 µS/cm bis 0,5 mS/cm aufweist und wobei die wässrige Lösung mit niedriger Leitfähigkeit einen pH-Wert von etwa 7 oder höher aufweist, und zusätzliches Waschen des Protein-A-Affinitätschromatographiematerials mit einer wässrigen Lösung mit mittlerer Leitfähigkeit vor oder nach dem Waschen des Protein-A-Affinitätschromatographiematerials mit einer wässrigen Lösung mit niedriger Leitfähigkeit, wobei die wässrige Lösung mit mittlerer Leitfähigkeit einen Leitfähigkeitswert von mehr als 0,5 mS/cm bis weniger als 20 mS/cm aufweist,
d) Gewinnen des bispezifischen Antikörpers aus dem Protein-A-Affinitätschromatographiematerial,
e) Durchführen eines weiteren Chromatographieschritts,
und dadurch Reinigen des bispezifischen Antikörpers.

3. Verfahren nach einem der Ansprüche 1 bis 2, wobei der weitere Chromatographieschritt nach dem Protein-A-Affinitätschromatographieschritt ein Ionenaustauschchromatographieschritt oder ein multimodaler Ionenaustauschchromatographieschritt ist.

4. Verfahren nach einem der Ansprüche 1 bis 3, wobei die wässrige Lösung mit niedriger Leitfähigkeit etwa 0,1 mM bis etwa 8 mM Tris umfasst.

5. Verfahren nach einem der Ansprüche 1 bis 3, wobei die wässrige Lösung mit niedriger Leitfähigkeit etwa 0,05 mM bis etwa 2 mM Kaliumphosphat umfasst.

6. Verfahren nach einem der Ansprüche 1 bis 5, wobei das Verfahren zusätzlich das Waschen des Protein-A-Affinitätschromatographiematerials mit einer wässrigen Lösung mit hoher Leitfähigkeit und mit einer wässrigen Lösung mit mittlerer Leitfähigkeit vor oder nach dem Waschen des Protein-A-Affinitätschromatographiematerials mit einer wässrigen Lösung mit niedriger Leitfähigkeit umfasst, wobei die wässrige Lösung mit hoher Leitfähigkeit einen Leitfähigkeitswert von etwa 20 mS/cm oder höher aufweist und wobei die wässrige Lösung mit mittlerer Leitfähigkeit einen Leitfähigkeitswert von mehr als 0,5 mS/cm bis weniger als 20 mS/cm aufweist.

7. Verfahren nach Anspruch 6, wobei die wässrige Lösung mit hoher oder mittlerer Leitfähigkeit Histidin umfasst.

8. Verfahren nach einem der Ansprüche 1 bis 7, wobei der bispezifische Antikörper ein Antikörper vom IgG1- oder IgG4-Isotyp ist.

9. Verfahren nach einem der Ansprüche 1 bis 8, wobei der bispezifische Antikörper ein bispezifischer Antikörper ist, umfassend a) die schwere Kette und die leichte Kette eines ersten Antikörpers voller Länge, der spezifisch an ein erstes Antigen bindet; und b) die schwere Kette und die leichte Kette eines zweiten Antikörpers voller Länge, der spezifisch an ein zweites Antigen bindet, wobei die konstanten Domänen CL und CH1 durch einander ersetzt sind.

10. Verfahren nach einem der Ansprüche 1 bis 9, wobei das erste Antigen humaner VEGF ist und das zweite Antigen humaner ANG-2 ist oder das erste Antigen humaner ANG-2 ist und das zweite Antigen humaner VEGF ist.

11. Verfahren nach einem der Ansprüche 1 bis 10, wobei die erste Antigenbindungsstelle als variable Domäne der schweren Kette (VH) die SEQ ID NO: 1 und als variable Domäne der leichten Kette (VL) die SEQ ID NO: 2 umfasst; und die zweite Antigenbindungsstelle als variable Domäne der schweren Kette (VH) die SEQ ID NO: 3 und als variable Domäne der leichten Kette (VL) die SEQ ID NO: 4 umfasst.

## Revendications

1. Procédé de production d'un anticorps bispécifique comprenant un premier site de liaison à l'antigène qui se lie spécifiquement à un premier antigène et un second site de liaison à l'antigène qui se lie spécifiquement à un second antigène comprenant les étapes suivantes
a) culture d'une cellule comprenant un acide nucléique codant pour l'anticorps bispécifique,
b) récupération de l'anticorps bispécifique de la cellule ou du milieu de culture,
c) mise en contact de l'anticorps bispécifique avec un matériau de chromatographie d'affinité sur protéine A,
d) lavage du matériau de chromatographie d'affinité sur protéine A avec une solution aqueuse de faible conductivité, la solution aqueuse de faible conductivité ayant une valeur de conductivité de 0,03 µS/cm à 0,5 mS/cm et la solution aqueuse de faible conductivité ayant un pH d'environ 7 ou plus, et de plus lavage du matériau de chromatographie d'affinité sur protéine A avec une solution aqueuse de conductivité moyenne avant ou après le lavage du matériau de chromatographie d'affinité sur protéine A avec une solution aqueuse de faible conductivité, la solution aqueuse de conductivité moyenne ayant une valeur de conductivité de plus de 0,5 mS/cm à moins de 20 mS/cm,
e) récupération de l'anticorps bispécifique du matériau de chromatographie d'affinité sur protéine A,
f) réalisation d'une étape de chromatographie supplémentaire,
et production ainsi de l'anticorps bispécifique.

2. Procédé de purification d'un anticorps bispécifique comprenant un premier site de liaison à l'antigène qui se lie spécifiquement à un premier antigène et un second site de liaison à l'antigène qui se lie spécifiquement à un second antigène à partir d'un échantillon comprenant les étapes de
a) fourniture d'un échantillon comprenant l'anticorps bispécifique,
b) application de l'échantillon comprenant l'anticorps bispécifique sur un matériau de chromatographie d'affinité sur protéine A,
c) lavage du matériau de chromatographie d'affinité sur protéine A avec une solution aqueuse de faible conductivité, la solution aqueuse de faible conductivité ayant une valeur de conductivité de 0,03 µS/cm à 0,5 mS/cm et la solution aqueuse de faible conductivité ayant un pH d'environ 7 ou plus, et de plus lavage du matériau de chromatographie d'affinité sur protéine A avec une solution aqueuse de conductivité moyenne avant ou après le lavage du matériau de chromatographie d'affinité sur protéine A avec une solution aqueuse de faible conductivité, la solution aqueuse de conductivité moyenne ayant une valeur de conductivité de plus de 0,5 mS/cm à moins de 20 mS/cm,
d) récupération de l'anticorps bispécifique du matériau de chromatographie d'affinité sur protéine A
e) réalisation d'une étape de chromatographie supplémentaire,
et ainsi purification de l'anticorps bispécifique.

3. Procédé selon l'une quelconque des revendications 1 à 2, dans lequel l'étape de chromatographie supplémentaire après l'étape de chromatographie d'affinité sur protéine A est une étape de chromatographie par échange d'ions ou une étape de chromatographie par échange d'ions multimodale.

4. Procédé selon l'une quelconque des revendications 1 à 3, dans lequel la solution aqueuse de faible conductivité comprend environ 0,1 mM à environ 8 mM de Tris.

5. Procédé selon l'une quelconque des revendications 1 à 3, dans lequel la solution aqueuse de faible conductivité comprend environ 0,05 mM à environ 2 mM de phosphate de potassium.

6. Procédé selon l'une quelconque des revendications 1 à 5, dans lequel le procédé comprend en outre le lavage du matériau de chromatographie d'affinité sur protéine A avec une solution aqueuse de conductivité élevée et avec une solution aqueuse de conductivité moyenne avant ou après le lavage du matériau de chromatographie d'affinité sur protéine A avec une solution aqueuse de faible conductivité, dans lequel la solution aqueuse de conductivité élevée a une valeur de conductivité d'environ 20 mS/cm ou plus et dans lequel la solution aqueuse de conductivité moyenne a une valeur de conductivité de plus de 0,5 mS/cm à moins de 20 mS/cm.

7. Procédé selon la revendication 6, dans lequel la solution aqueuse de conductivité élevée ou moyenne comprend de l'histidine.

8. Procédé selon l'une quelconque des revendications 1 à 7, dans lequel l'anticorps bispécifique est un anticorps d'isotype IgG1 ou IgG4.

9. Procédé selon l'une quelconque des revendications 1 à 8, dans lequel l'anticorps bispécifique est un anticorps bispécifique comprenant a) la chaîne lourde et la chaîne légère d'un premier anticorps pleine longueur qui se lie spécifiquement à un premier antigène ; et b) la chaîne lourde et la chaîne légère d'un second anticorps pleine longueur qui se lie spécifiquement à un second antigène, dans lequel les domaines constants CL et CH1 sont remplacés l'un par l'autre.

10. Procédé selon l'une quelconque des revendications 1 à 9, dans lequel le premier antigène est le VEGF humain et le second antigène est l'ANG-2 humaine ou le premier antigène est l'ANG-2 humaine et le second antigène est le VEGF humain.

11. Procédé selon l'une quelconque des revendications 1 à 10, dans lequel ledit premier site de liaison à l'antigène comprend en tant que domaine variable de chaîne lourde (VH) la SEQ ID NO : 1, et en tant que domaine variable de chaîne légère (VL) la SEQ ID NO : 2 ; et ledit second site de liaison à l'antigène comprend en tant que domaine variable de chaîne lourde (VH) la SEQ ID NO : 3, et en tant que domaine variable de chaîne légère (VL) la SEQ ID NO : 4.
